# EUROPEAN PATENT SPECIFICATION

(11) **EP 1 006 121 B1**
(45) Date of publication and mention of the grant of the patent: **02.05.2003**
(21) Application number: 98309881.5
(22) Date of filing: 02.12.1998
(51) Int. Cl.: C07H 21/00, C03C 14/00, C07C 31/20

(54) **A universal polymer support for the synthesis of oligonucleotides**
Universale Polymere Träger zur Synthese von Oligonukleotiden
Support polymère universel pour la synthèse des oligonucléotides

(43) Date of publication of application: 07.06.2000
(73) Proprietor: Council of Scientific and Industrial Research, New Delhi 110 001 (IN)
(72) Inventor: Kumar, Pradeep, Delhi 110007 (IN); Gupta, Kailash Chand, Delhi 110007 (IN)
(74) Representative: Smart, Peter John

(56) References cited:
- WO-A-95/01987
- WO-A-97/40458
- CA-A- 1 336 690
- US-A- 5 451 463
- US-A- 5 539 097
- US-A- 5 601 979
- SCHWARTZ M E ET AL: "A Universal Adapter for Chemical Synthesis of DNA or RNA on any Single Type of Solid Support" TETRAHEDRON LETTERS, vol. 36, no. 1, 2 January 1995, page 27-30 XP004028936

## Description

### FIELD OF THE INVENTION

The present invention relates to of an improved universal polymer support useful for the synthesis of oligodeoxyribo- and oligoribonucleotides and a process for preparing such universal polymer support.

The improved universal polymer support of the present invention consists of a polymer support containing an organic aliphatic molecule having at least a pair of cis-hydroxyl groups on adjacent carbons where one of the two hydroxyl groups is attached to the polymer support through a covalent linkage and the other hydroxyl group is protected with an acid labile group.

The improved universal polymer support consists of an organic or inorganic polymer material bearing aminoalkyl or hydroxyl functionalities and an organic aliphatic substance bearing at least a pair of cis-hydroxyl groups (both secondary) on adjacent carbons where one of the hydroxyl functions is coupled to aminoalkyl or hydroxyl function via a succinate linkage. The other hydroxyl function of the organic substance was protected with a substituted trityl group using monomethoxytrityl or 4,4'-dimethoxytrityl chloride.

### PRIOR ART REFERENCES

The known protecting groups employed for the protection of exocyclic amino functionalities of nucleic bases are as defined below:

### Set I

### Conventional protecting groups

B = Guanine; R = Isobutyryl (ibu)
B = Cytosine; R = Benzoyl (bz) or Acetyl (ac)

### Set II

### Labile protecting groups

B = Guanine; R = Isopropylphenoxyacetyl (IPac)
B = Cytosine; R = Isobutyryl (ibu) or Acetyl (Ac)
B = Adenine; R = Phenoxyacetyl (Pac)

### Set III

### Labile protecting groups

B = Guanine, Cytosine, Adenine; R = Dimethylformamidine (Dmf)

### Set IV

### Labile protecting groups

B = Guanine, Cytosine, Adenine; R = p-tert-Butylphenoxyacetyl

Synthetic oligonucleotides (oligodeoxyribo- and oligoribonucleotide) have become indispensable tools in modern biological sciences. The protocols for chemical synthesis and modifications of these molecules have been simplified to an extent that even a non-chemist, the actual user can synthesize these molecules without much difficulties. However, the support functionalization has been one of the time consuming tasks. Moreover, one still requires to use at least eight different (four for oligodeoxyribo- and four for oligoribonucleotides) supports, each bearing a separate nucleoside corresponding to the 3'-terminus of the desired oligomer. A number of base labile group containing phosphoramidite synthons have been proposed and used (shown in sets II-IV) to speed up the post synthesis (deprotection) time. Therefore, one would require to prepare the pre-function-alized supports accordingly and hence the number of pre-derivatized supports would be very large. This number may even be more when 3'-terminus of the desired oligomer contains nucleotide other than the normal one. In the past few years, some attempts have been made to develop universal supports to eliminate the use of at least eight different supports. Gough et al. (Tet. Lett. 24, 1983, 5321) have proposed the use of a universal support that consisted of controlled pore glass derivatized with 2'(3')-O-benzoyluridine-5'-O-succinyl residue and demonstrated its usefulness for the synthesis of oligodeoxyribo- and oligoribonucleotides via phosphotriester or the phosphite approach. The support contains a nucleosidic material which does not get incorporated in the oligonucleotide chain and goes waste. Further, the functionalization of the support involves multisteps (4 steps) synthesis and purification procedure. In a more recent publication, Gough and co-workers (Tet. Lett., 36, 1995, 27) have further modified the functinalization of the universal support by the use of an adaptor, 2' (3')-O-benzoyluridine-5'-O-(cyanoethylN,N-diisopropyl-phosphoramidite) and coupled it to a standard T-support, following phosphoramidite chemistry. The functionalized support was subsequently used for the synthesis of oligonucleotides.

However, the universal support has still the following serious limitations. The support contains two nucleosidic units instead of one in earlier support (very expensive material) which do not get incorporated in the oligonucleotide chain and go waste. The adaptor used for the functionalization of universal support involves multistep synthesis and purification strategy and, hence, the yield of the final product is low, making the support functionalization a time consuming (2-3d), tedious and expensive. Further, the deprotection time of oligodeoxyribo- and oligoribonucleotides synthesized using base labile group containing nucleosidephosphoramidite synthons is very small e.g. 20 min in aq. ammonia at 60°C or 1-2h in aq. ammonia at room temperature. These requirements are not met by the universal supports proposed by Gough et al.(Tet. Lett., 24, 1983, 5321; ibid. 36, 1995, 27).

Recently, one more universal support (Nucl. Acids Res, 1996, 24, 2793) containing a 1-O-(4,4'-dimethoxytrityl)-2-O-succinyl-3-N-allyloxycarbonylpropane immobilized on aminopropyl-CPG has been proposed. However, the release of oligomers from the support involves three hectic steps.

Therefore, there is a need to develop a universal polymer support suitable for the synthesis of oligodeoxyribo- and oligoribonucleotides and compatible to the existing methods of their synthesis and deprotection using nucleosidephosphoramidite synthons carrying conventional or base labile protecting groups for exocyclic amino functionalities.

Further, there is also a need for to provide a process for the preparation of an improved universal polymer support devoid of any nucleosidic material, suitable for the synthesis of all possible oligodeoxyribo- and oligoribonucleotides.

Furthermore, there is a need to provide a process for the preparation of an improved universal polymer support compatible to the existing methods of oligonucleotide synthesis and deprotection using nucleosidephosphoramidite synthons carrying conventional or base labile protecting groups for exocyclic amino functionalities.

Keeping the above needs in view, the applicants designed some supports of the structures shown below: 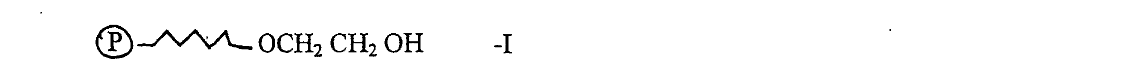 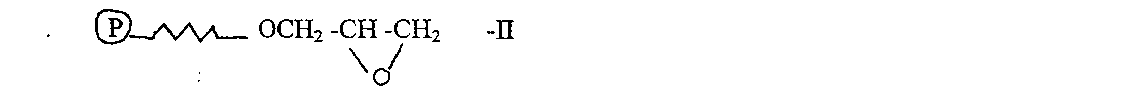 The supports I and II were found suitable for the synthesis of oligodeoxyribo- and oligoribonucleotides using nucleosidephosphoramidite synthons carrying conventional and base labile protecting groups for exocyclic amino functionalities as shown in the above mentioned sets I-IV, but the oligonucleotides did not cleave at all from the support I with aq. ammonia in 24h at 60°C and hence not suitable for oligonucleotide synthesis. In case of support II, no cleavage occured under normal deprotection conditions whereas only 90% oligonucleotide cleaved with aq. ammonia at very high temperature (100°C) and the remaining amount goes waste, which is not desirable in oligonucleotide synthesis (WO 95/01987). In addition, this patent application (WO 95/01987), described a universal polymer support where glycol type of moiety has been employed with adjacent alkyl groups substituted by one or more halogen(s). These halogen groups present in alkyl substitution prevent the free rotation of hydroxyl groups and hence slow down the formation of cyclic phosphates during deprotection process under mild conditions. Moreover, the complete cleavage of oligonucleotides require harsh conditions, which are not at all desirable for some base sensitive oligonucleotides. Such types of supports are not compatible with the deprotection conditions-required for the deprotection of oligonucleotides synthesized using nucleosidephosphoramidite synthons carrying base Labile protection (Set II-IV) for nucleic bases. Furthermore, such supports can not be employed where deprotection is to be carried out in 5-20 min (Table-1 given below) .

In search of a suitable mechanism for the rapid cleavage of oligonucleotides under mild conditions (Table 1), we noticed that Gough and co-workers had already reported that a nucleotide having 3'-terminal protection with a ribonucleoside linked by its 2'- or 3'-hydroxyl group to a 3'-phosphate is removed as its 2',3'-cyclic phosphate by lead cation.

In this process a nucleotide or oligonucleotide gets removed with 3'-hydroxyl function. This concept led Gough and co-workers to develop two universal supports (Gough et al., Tet. Lett., 24, 1983, 5321; ibid. 36, 1995, 27) useful for the preparation of oligonucleotides. Because of the rigid rotation of the hydroxyl groups in uridine, oligodeoxyribo- and oligoribonucleotides take considerable time (48h) for cleaving from the universal supports. Similarly the supports prepared using non-nucleosidic linker e.g. using polymer supported 1,4-anhydroerythritol (Clontech, USA) also not compatible to the deprotection conditions required for the cleavage of oligonucleotides synthesized using base labile nucleosidephosphoramidite synthons.

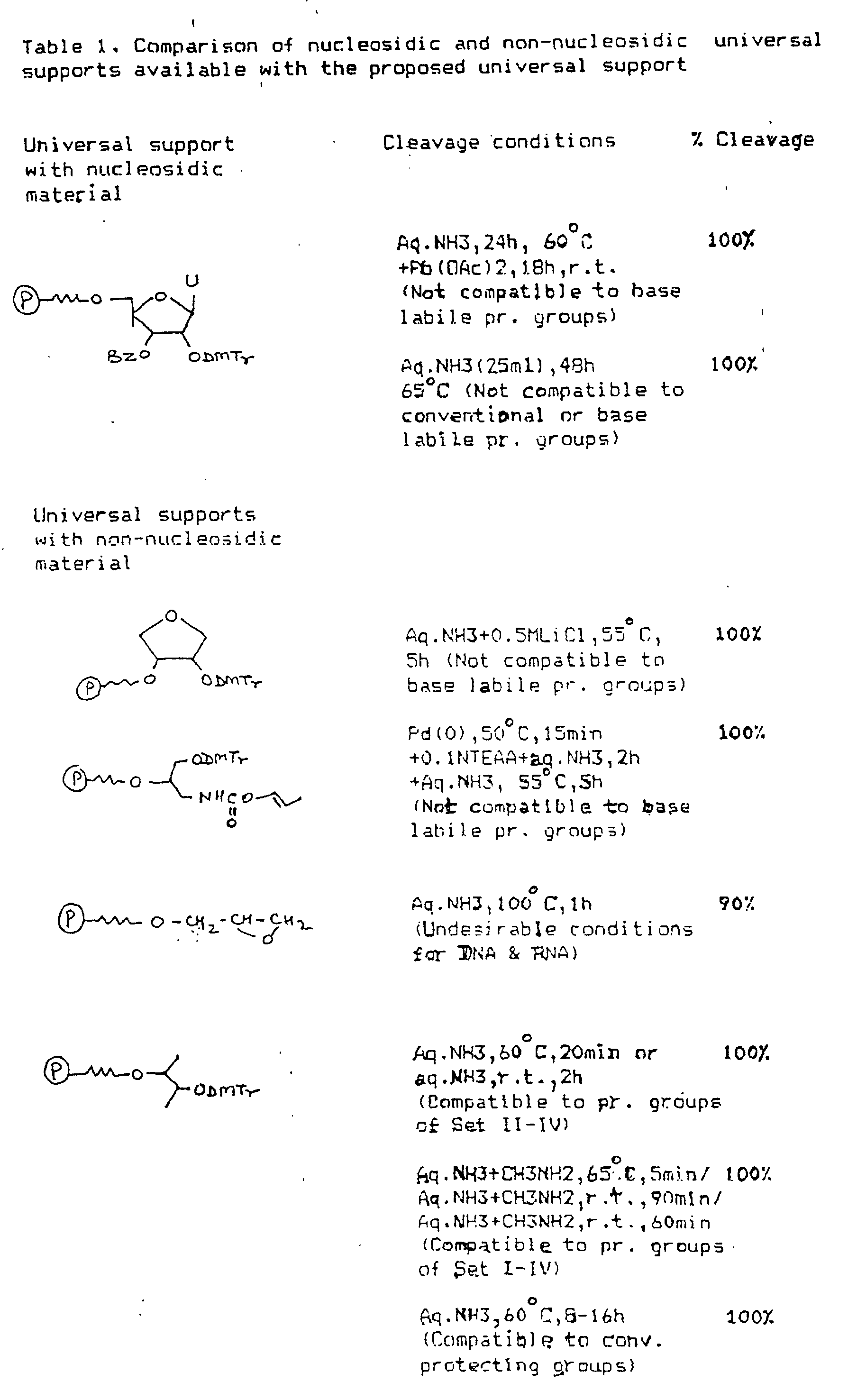

The above envisaged problems might be taken care of if the source of cis diol groups is replaced by an organic molecule having a pair of cis hydroxyl groups with free rotation. The free rotation of vicinal hydroxyl groups will help in rapid formation of cyclic phosphate under mild alkaline conditions and hence will lead to rapid deprotection of oligodeoxyribo- and oligoribonucleotides from an improved universal polymer support.

### OBJECTS OF THE INVENTION

Accordingly, the main object of the invention is, therefore, to provide of an improved universal polymer support useful for the synthesis of all possible oligdeoxyribo- and oligoribonucleotides.

Another object of the present invention is to provide a process for the preparation of an improved universal polymer support devoid of any nuleosidic material, suitable for the synthesis of all possible oligodeoxyribo- and oligoribonucleotides.

Still another object of the present invention is to provide a process for the preparation of an improved universal polymer support compatible to the existing methods of oligonuceotide synthesis and deprotection using nucleosidephoosphoramidite synthons carrying conventional or base labile protecting groups for exocyclic amino functionalities.

### SUMMARY OF THE INVENTION

Based on the these facts, the applicants have now proposed the following molecule for the preparation of a universal support: 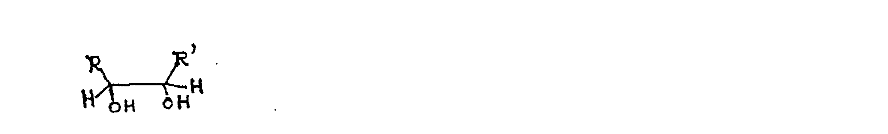 R= H-(CH₂)ₙ- and R'= -CH₂-OH, -(CH₂)ₙ-H; n = 1-4, and a process for preparing the said universal support.

### DETAILED DESCRIPTION OF THE INVENTION

In addition to the above, present invention provides a process for the preparation of a universal polymer support of thestructure provided with the specifications, devoid of any nucleosidic material useful for the synthesis of oligodeoxyribo-and oligoribonucleotides, which comprises:
(i) treating an organic aliphatic molecule having a pair of hydroxyl groups (both are at secondary position) at adjacent carbon atoms with 4,4'-dimethoxytrityl chloride and isolating the monosubstituted substance 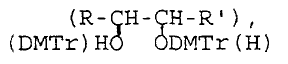 where R = H-(CH₂)ₙ- and R' = -CH₂-OH, -(CH₂)ₙ-H; n = 1-4; DMTr = 4,4'-dimethoxytrityl.
(ii) treating the monosubstituted substance obtained in step (i) with one equivalent of a homobifunctional alkanoic acid halide and transfering the mixture to a polymer support carrying hydroxyl or aminoalkyl functionalities.
(iii) treating the polymer support obtained in step (ii) with dry alkanol for blocking the residual functional groups (capping) on the universal polymer support followed by washing with dry alkanol and dialkyl ether, respectively.

The organic aliphatic molecule having a pair of hydroxyl groups (both are at secondary positions) on adjacent carbons used for making monosubstituted tritylated derivative may be selected from butane-2,3-diol, 1,2,3-trihydroxyheptane, 1,2,3-hexanetriol and the like.

The homobifunctional alkanoic acid halide used in step (ii) may be selected from oxalyl chloride, succinoyl chloride, adipoyl chloride and the like.

The polymer support employed in step (ii) may be selected from organic or inorganic polymers e.g. controlled pore glass (CPG) with variable pore size and linker arm, silica gel (porous or non-porous), cross-linked polystyrene having hydroxyl or aminoalkyl functions and the like.

The alkanol used in step (iii) for capping purpose may be selected from methanol, ethanol, propanol and the like.

One embodiment of the invention relates to a process for the preparation of oligonucleotides (both oligodeocyribo-and oligoribo-) using an improved universal polymer support which is compatible to the existing method of oligonucleotide synthesis and deprotection using nucleosidephosphoramidite synthons carrying conventional or base labile protecting groups for the protection of exocyclic amino functionalities of the nucleic bases.

Another embodiment of the invention relates to a preparation of oligonucleotides using an improved universal support comprises:
(i) synthesising oligodeoxyribo- and oligoribonucleotides on an improved universal polymer support following phosphoramidite chemistry employing conventional or labile protecting groups for nucleic bases,
(ii) treating the oligodeoxynucleotide and oligoribonucleotides embeded in the support with a basic medium,
(iii) concentrating the reaction residue obtained in step (ii) in a vacuum concentrator,
(iv) dissolving the solid residue obtained in step (iii) in water and subjecting to gel filtration to remove low molecular weight impurities, and
(v) concentrating the eluent obtained in step (iv) under vacuum concentrator and subjecting to high performance liquid chromatography (HPLC) to obtain the free fully deprotected oligodeoxyribo and oligoribonucleotides.

The 2'-deoxyribonucleosidephosphoramidites and ribonucleosidephosphor-amidites used for the synthesis of oligodeoxyribo-and ribonucleotides in step (i) carrying conventional protecting groups are selected as follows benzoyl for adenine, benzoyl or acetyl for cytosine and isobutyryl for guanine or labile protecting groups are selected from phenoxyacetyl for adenine and guanine and isobutyryl or acetyl for cytosine; dimethylformamidine (DMF) for adenine, guanine and cytosine; p-tert-butylphenoxyacetyl- for adenine, guanine and cytosine and the like for exocyclic amino functions of nucleic bases.

In the present invention, the basic medium used for cleaving synthesized oligonucleotides from the universal polymer support and simultaneous removal of protecting groups is selected from aq. Ammonia, ag. methylamine, ag. ammonia - methylamine (1:1,v/v) mixture. The oligomers are cleaved from the universal polymer support and the protecting groups are simultaneously removed at a temperature in the range of 30° -65° C depending upon the protecting groups employed for the protection of exocyclic amino groups of nucleic bases, and the time required for cleaving oligomers from the universal polymer support and simultaneous removal of protecting groups is in the range of 5-120 min depending upon the protecting groups employed for the protection of exocyclic amino groups of nucleic bases.

The following examples concerning the preparation of universal polymer support useful for the synthesis of oligodeoxynucleotides and oligoribonucleotides are provided to illustrate the invention and should not be construed to limit the scope of the invention.

### Example 1

The preparation of universal support comprises of two steps which are described below. In a 25 ml round bottomed flask, dibutan-2 ,3-diol (5 mmol) was dried by coevaporation with pyridine (15 ml) and finally suspended in anhydrous pyridine (15 ml). 4,4' -Dimethoxytrityl chloride (2 mmol) was added and the reaction mixture was left for stirring at room temperature. After 5h, 5% aq. Sodium hydrogen carbonate (2 ml) was added and stirred for further 10 min. Then the reaction mixture was concentrated on a rotary evaporator and the syrupy material so obtained was redissolved in ethyl acetate (70 ml) and washed with aq. Sodium hydrogen carbonate solution (2 x 25ml) and water (1 x 25 ml), respecttively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol, in 90% yield and fully characterized by its NMR, IR and MS spectra.

In the second step, 2-O-(4,4'-dimethoxytrityl)-butan-2,3-diol was attached to the long chain aminoalkylated controlled pore glass (LCAA-CPG) by following procedure. Succinoyl chloride (6.3 ul, 0.05 mmol) was added to a solution of triazole (19.8 mg, 0.28 mmol) in an anhydrous mixture of pyridine (500 ul) and acetonitrile (1.0 ml) taken in a septum sealed vial. To the solution was injected 2-O-(4,4'-dimethoxytrityl)-butan-2,3-diol (0.05 mmol) in acetonitrile (1 ml). The reaction mixture was agitated at room temperature for 1h and then LCAA-CPG (200 mg) added and agitation continued at room temperature. After 15 min, the contents of the vial were transferred to sintered disc glass funnel, excess reagent washed off, residual triazolosuccinoyl groups on the polymer support were capped with anhydrous methanol (1 ml). After washing with dry acetonitrile (3 x 5 ml), residual amino groups were blocked following standard procedure (Matteucci, M.D. and Caruthers, M.H., J.Am.Chem.Soc., 103, 1981, 3185).

### Example 2

In a 25 ml round bottomed flask, d-butan-2,3-diol (5 mmol) was dried by coevaporation with pyridine (15 ml) and finally suspended in anhydrous pyridine (15 ml). 4,4'-Dimethoxytrityl chloride (2 mmol) was added and the reaction mixture was left for stirring at room temerature. After 5h, 5% aq. sodium hydrogen carbonate (2 ml) was added and stirred for further 10 min. Then the reaction mixture was concentrated on a rotary evaporator and the syrupy material so obtained was redissolved in ethyl acetate (70 ml) and washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol, in 85% yield and fully characterized by its NMR, IR and MS spectra.

The compound, 2-O-(4,4'-dimethoxytrityl)-butan-2,3-diol, (0.1 mmol) was reacted with succinic anhydride (0.2 mmol) in dichloroethane (4 ml) in the presence of 4-dimethylaminopyridine (0.05 mmol) and a tertiary base, triethylamine (0.1 mmol) at 50°C for 20 min. After stipulated time (20 min), the reaction mixture was diluted with dichloroethane (25 ml) and washed with 5% cold aq. citric acid (2 x 15 ml) and water (2 x 10 ml), respectively. The organic phase was collected and concentrated to obtain 3-(4,4'-dimethoxytrityloxy)-2-butylsuccinate in 93% yield. 3-(4,4'-dimethoxytrityloxy)-2-butylsuccinate (0.05 mmol) and 4-dimethylaminopyridine (0.01 mmol) were dissolved in pyridine (2 ml) and added LCAA-CPG (200 mg) followed by dicyclohexylcarbodiimide (0.075 mmol) under anhydrous conditions. The resulting mixture was shaken occasionally at room temperature for 16h. Then the mixture was transferred to a sintered disc glass funnel and washed off the excess reagents with pyridine (1 x 10 ml), methanol (3 x 15 ml) and diethylether (2 x 10 ml). The washed support was dried under vacuum, capped the residual amino groups on the polymer support following standard procedure (Matteucci, M.D. and Caruthers, M.H., J.Am.Chem.Soc., 103, 1981, 3185).

### Example 3

In a 25 ml round bottomed flask, l-butan-2,3-diol (5 mmol) was dried by coevaporation with pyridine (15 ml) and finally suspended in anhydrous pyridine (15 ml). 4,4'-Dimethoxytrityl chloride (2 mmol) was added and the reaction mixture was left for stirring at room temperature. After 5h, 5% aq. sodium hydrogen carbonate (2 ml) was added and stirred for further 10 min. Then the reaction mixture was concentrated on a rotary evaporator and the syrupy material so obtained was redissolved in ethyl acetate (70 ml) and washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol, in 88% yield and fully characterized by its NMR, IR and MS spectra.

In the next reaction, 2-O-(4,4'-dimethoxytrityl)butan-2,3-diol, (0.1 mmol) was reacted with succinic anhydride (0.2 mmol) in dichloroethane (4 ml) in the presence of 4-dimethylaminopyridine (0.05 mmol) and a tertiary base, triethylamine (0.1 mmol) at 50°C for 20 min. After stipulated time, the reaction mixture was diluted with dichloroethane (25 ml) and washed with 5% cold aq. citric acid (2 x 15 ml) and water (2 x 10 ml), respectively. The organic phase was collected and concentrated to obtain 3-(4,4'-dimethoxytrityloxy)-2-butylsuccinate in 93% yield. To the mixture of 3-(4,4'-dimethoxytrityloxy)-2-butylsuccinate (0.05 mmol), 2,2'-dithiobis(5-nitropyridine) (DTNP) (0.05 mmol) and 4-dimethylaminopyridine (0.05 mmol) dissolved in acetonitrile : dichloroethane (3:1, v/v, 2 ml) was added a solution of triphenylphosphine (0.05 mmol) in acetonitrile (0.5 ml) followed by addition of LCAA-CPG (250 mg). The resulting mixture was shaken occasionally at room temperature for 30 min. Then the mixture was transferred to a sintered disc glass funnel and washed off the excess reagents with acetonitrile (1 x 10 ml), dichloroethane (3 x 10 ml), methanol (3 x 15 ml) and diethylether (2 x 10 ml). The washed support was dried under vacuum and blocked the residual amino groups on the polymer support with acetic anhydride (Matteucci, M.D. and Caruthers, M.H., J.Am.Chem.Soc., 103, 1981, 3185).

### Example 4

In a 25 ml round bottomed flask, d-butan-2,3-diol (5 mmol) was dried by coevaporation with pyridine (15 ml) and finally suspended in anhydrous pyridine (15 ml). 4,4'-Dimethoxytrityl chloride (2 mmol) was added and the reaction mixture was left for stirring at room temerature. After 5h, 5% aq. sodium hydrogen carbonate (2 ml) was added and stirred for further 10 min. Then the reaction mixture was concentrated on a rotary evaporator and the syrupy material so obtained was redissolved in ethyl acetate (70 ml) and washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol, in 85% yield and fully characterized by its NMR, IR and MS spectra.

The next step was carried out with slight modification. LCAA-CPG (500 mg) was activated with 3% trichloracetic acid (20 ml) in dichloromethane for 3h at room temperature followed by washings with dichloroethane (2 x 50 ml), triethylamine : diisopropylethylamine (1:1, v/v, 50 ml) and diethylether (1 x 25 ml). Then the support was dried in a vacuum desiccator. To the suspension of LCAA-CPG (250 mg) in dry dichloroethane (3 ml) was added succinic anhydride (0.25 mmol), triethylamine (0.05 mmol) and 4-dimethylaminopyridine (0.025 mmol) for 5h at 55°C. Then it was filtered and excess of reagents were removed with dichloroethane (3 x 20 ml), methanol (2 x 20 ml) and diethylether (2 x 15 ml) followed by drying under vacuum. 2-O-(4,4'-dimethoxytrityl)-butan-2,3-diol (0.05 mmol) was mixed with 4-dimethylaminopyridine (0.05 mmol), 1-(3-dimethylaminopropyl)-ethylcarbodiimide (0.25 mmol) and succinylated LCAA-CPG (200 mg) in pyridine (2 ml) and triethylamine (30 (mol). The flask was tightly sealed and shaken at room temperature for 16h. Pentafluorophenol (0.05 mmol) was added and shaking was continued for another 16h. Then piperidine (500 umol) was added, shaken for 5 min and filtered off the support. It was washed with dichloromethane (2 x 20 ml) and diethylether (2 x 10 ml). After drying under vacuum, the residual amino groups were capped with acetic anhydride to obtain the universal polymer support.

### Example 5

In a 25 ml round bottomed flask, dl-butan-2,3-diol (5 mmol) was dried by coevaporation with pyridine (15 ml) and finally suspended in anhydrous dichloroethane (15 ml). 4,4'-Dimethoxytrityl chloride (2 mmol) was added and the reaction mixture was left for stirring at room temerature. After 5h, 5% aq. sodium hydrogen carbonate (2 ml) was added and stirred for further 10 min. Then the reaction mixture was washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol,in 87% yield and fully characterized by its NMR, IR and MS spectra.

After accomplishing the first step, the second step was carried out as follows. Oxalyl chloride (8.7 ul, 0.1 mmol) was added to a solution of triazole (35 mg, 0.5 mmol) in an anhydrous mixture of pyridine (1 ml) and acetonitrile (5.0 ml) taken in a septum sealed vial. To the solution was added 2-O-(4,4'-dimethoxytrityl)-butan-2,3-diol (0.1 mmol) in acetonitrile (3ml). The reaction mixture was agitated at room temperature for 1h and then LCAA-CPG (300 mg) added and agitation continued at room temperature. After 15 min, the contents of the vial were transferred to sintered disc glass funnel, excess reagent washed off, residual triazolo-oxalyl groups on the polymer support were capped with anhydrous methanol (5 ml). After washing with dry acetonitrile (3 x 25 ml), the residual amino groups were blocked to obtain the universal support.

### Example 6

In the first step, l-butan-2,3-diol (5 mmol) was dried by coevaporation with pyridine (15 ml) and finally suspended in anhydrous acetonitrile (15 ml). 4,4'-Dimethoxytrityl chloride (2 mmol) was added and the reaction mixture was left for stirring at room temerature. After 5h, 5% aq. sodium hydrogen carbonate (2 ml) was added and stirred for further 10 min. Then the reaction mixture was concentrated on a rotary evaporator and the syrupy material so obtained was redissolved in ethyl acetate (70 ml) and washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vaccum) to obtain, 2-O-(4,4'-dimethoxytrityl)-butan-2,3-diol, in 86% yield and fully characterized by its NMR, IR and MS spectra.

After accomplishing the first step, succinoyl chloride (8.7 ul, 0.1 mmol) was added to a solution of triazole (35 mg, 0.5 mmol) in an anhydrous mixture of pyridine (1 ml) and acetonitrile (5.0 ml) taken in a septum sealed vial. To the solution was added 2-O-(4,4'-dimethoxytrityl)-butan-2,3-diol (0.1 mmol) in acetonitrile (3 ml). The reaction mixture was agitated at room temperature for 1h and then LCAA-CPG (300 mg) added and agitation continued at room temperature. After 15 min., the contents of the vial were transferred to sintered disc glass funnel, excess reagent washed off, residual triazolosuccinoyl groups on the polymer support were capped with anhydrous methanol (5 ml). After washing with dry acetonitrile (3 x 25 ml), residual amino groups were blocked to obtain the universal support.

### Example 7

In a 25 ml round bottomed flask, 1,2,3-trihydroxyheptane (5 mmol) was dried by coevaporation with pyridine (15 ml) and finally suspended in anhydrous dimethylformamide (15 ml). t-Butyldimethylsilyl chloride (5.5 mmol) and imidazole (11 mmol) were added and the reaction mixture was allowed to stir for 6h at room temperature. After completion, the solvent was removed, the residue was dissolved in dichloroethane (50 mol) and washed with 10% aq. citric acid and water (50 ml each). The organic phase was collected and concentrated under reduced pressure to a syrupy material, 1-O-t-butyldimethylsilyl-1,2,3-trihydroxyheptane (4.2 mmol, 84%). In the second step, 1-O-t-butyldimethylsilyl-1,2,3-trihydroxyheptane (4 mmol) was dried with anhydrous pyridine (2 x 30 ml) and finally dissolved in 25 ml of pyridine, 4,4'-dimethoxytrityl chloride (5 mmol) was added and the reaction mixture was left for stirring at room temerature. After 5h, acetic anhydride (20 mmol), 4-dimethylaminopyridine (0.5 mmol) were added and the mixture stirred for 1h. Then the reaction was quenched by the addition of 5% aq. sodium hydrogen carbonate (2 ml) and the reaction mixture concentrated on a rotary evaporator to obtain a syrupy material which was redissolved in ethyl acetate (70 ml) and washed with aq. sodium hydrogen carbonate solution (2 x 25 ml) and water (1 x 25 ml), respectively. The organic layer was collected, dried over anhydrous sodium sulfate, concentrated and dried on an evaporator under reduced pressure(under vacum) to obtain, 1-O-t-butyldimethylsilyl-2(3)-O-4,4'-dimethoxytrityl, 3(2)-O-acetyl-1,2,3-trihydroxyheptane, in 70% yield. The compound was fully characterized by its NMR, IR and MS spectra.

The compound, 1-O-t-butyldimethylsilyl-2(3)-O-4,4'-dimethoxytrityl-3(2)-O-acetyl-1,2,3-trihydroxyheptane (1.5 mmol), was then treated with tetrabutylammonium fluoride (30 mmol) in dry tetrahydrofuran for 24h at room temperature to obtain 2(3)-O-4,4'-dimethoxytrityl-3(2)-O-acetyl-1,2,3-trihydroxyheptane in 85% yield.

The next step was carried out with slight modification. LCAA-CPG (500 mg) was activated with 3% trichloracetic acid (20 ml) in dichloromethane for 3h at room temperature followed by washings with dichloroethane (2 x 50 ml), triethylamine : diisopropylethylamine (1 : 1, v/v, 50 ml) and diethylether (1 x 25 ml). Then the support was dried in a vacuum desiccator. To the suspension of LCAA-CPG (250 mg) in dry dichloroethane (3 ml) was added succinic anhydride (0.25 mmol), triethylamine (0.05 mmol) and 4-dimethylaminopyridine (0.025 mmol) for 5h at 55°C. Then it was filtered and excess of reagents were removed with dichloroethane (3 x 20 ml), methanol (2 x 20 ml) and diethylether (2 x 15 ml) followed by drying under vacuum. 2(3)-O-4,4'-dimethoxytrityl-3(2)-O-acetyl-1,2,3-trihydroxyheptane (0.05 mmol) was mixed with 4-dimethylaminopyridine (0.05 mmol), 1-(3-dimethylamino- propyl)-ethylcarbodiimide (0.25 mmol) and succinylated LCAA-CPG (200 mg) in pyridine (2 ml) and triethylamine (30 (mol). The flask was tightly sealed and shaken at room temperature for 16h. Pentafluorophenol (0.05 mmol) was added and shaking was continued for another 16h. Then piperidine (500 (mol) was added, shaken for 5 min and filtered off the support. It was washed with dichloromethane (2 x 20 ml) and diethylether (2 x 10 ml). After drying under vacuum, the residual amino groups were capped with acetic anhydride to obtain the universal support.

### Example 8

The polymer support obtained in Example 1 was employed for oligonucleotide synthesis, d(AGTC), using phosphoramidite approach. After synthesis, the oligonucleotide was released from the support and protecting groups removed using concentrated aq. ammonia (2 ml) at 60°C for 20 min. After usual work-up, the analysis was carried out on HPLC and compared with standard oligomer. They were found to be comparable in their retention time and UV pattern.

Characterization of 2-O-(4,4'-dimethoxytrityl)-butan-2-3,-diol
¹H NMR, (CDCl3)δ: 0.9-1.28(m, 6H), 3.25(m,1H), 3.6 (m, 1H) , 3.8 (s, 6H) 6.75-7.5 (m, 13H)
MS :393 (M⁺ )
IR,υ(cm⁻¹ ): 3250,3050,2870,1240,1120,855

### Advantages of the invention:

1. The process is rapid and economical.
2. The preparation of improved universal support involves commercially available reagents.
3. The process does not require multistep synthesis and purification protocol.
4. The improved universal support obviates the use of nucleosidic material.
5. The improved universal support is compatible to the existing methods of oligonucleotide synthesis and deprotection.

## Claims

1. A universal polymer support containing an organic aliphatic molecule structure 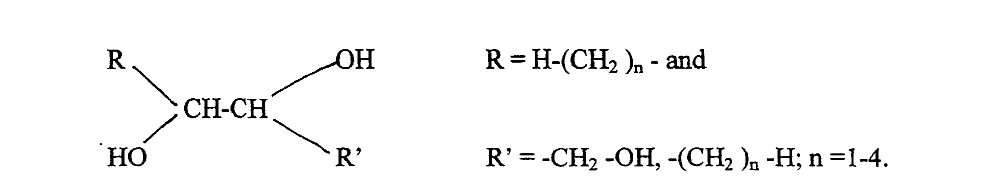 having at least a pair of cis-hydroxyl groups where one of the hydroxyl groups is attached to the polymer support through a covalent linkage and the other hydroxyl group is protected by an acid labile group.

2. A process useful for the preparation of fully deprotected oligonucleotides (deoxyribo- or ribo) which comprises:
(i) treating an aliphatic organic molecule having a pair of cis-hydroxyl groups at the secondary position with 4,4' -dimethoxytrityl chloride and isolating a monosubstituted tritylated derivative of the formula 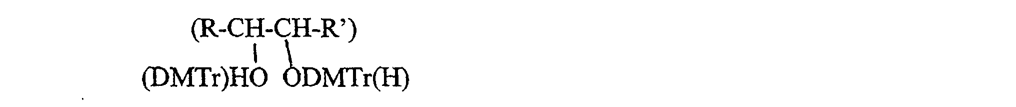 where R = H -(CH₂ )ₙ - and R' =⁻CH₂ OH, - (CH₂ )ₙ -H; n=1-4; DMTr = 4,4'-dimethoxytrityl,
(ii) treating the monosubstituted substance obtained in step (i) with one equivalent of a homobifunctional alkanoic acid halide and transferring the mixture to a polymer support carrying hydroxyl or aminoalkyl functionalities, and
(iii) treating the polymer support obtained in step (ii) with dry alkanol for capping purpose followed by washing with dry aklanol and dialkyl ether, respectively.

3. A process as claimed in claim 2 wherein the organic aliphatic molecule having a pair of cis-hydroxyl groups used for making the monosubstituted tritylated derivative is selected from the group consisting of butane-2-3- diol, 1,2,3-trihydroxyheptane and 1,2,3-hexanetriol.

4. A process as claimed claim 2 wherein the homobifunctional alkanoic acid halide used in step (ii) is selected from the group consisting of oxalyl chloride, succinoyl chloride, adipoyl chloride and the like.

5. A process as claimed in claim 2 wherein the polymer support employed in step (ii) is selected from organic or inorganic polymers e.g. controlled pore glass (CPG) with variable pore size and linker arm, silica gel (porous or non-porous), cross-linked polystyrene having hydroxyl or aminoalkyl functions and the like.

6. A process as claimed in claim 2 wherein the alkanol used in step (iii) for capping purpose is selected from methanol, ethanol and the like.

7. A process for the preparation of oligonucleotides (both oligodeocyribo-and oligoribo-) using an improved universal polymer support of claim 1 compatible to the existing method of oligonucleotide synthesis and deprotection using nucleosidephosphoramidite synthons carrying conventional or base labile protecting groups for the protection of exocyclic amino functionalities of the nucleic bases.

8. A process as claimed in claim 7 wherein the preparation of oligonucleotides using an improved universal support comprises:
(i) synthesising oligodeoxyribo- and oligoribonucleotides on an improved universal polymer support following phosphoramidite chemistry employing conventional or labile protecting groups for nucleic bases,
(ii) treating the oligodeoxynucleotide and oligoribonucleotides embedded in the support with a basic medium,
(iii) concentrating the reaction residue obtained in step (ii) in a vacuum concentrator,
(iv) dissolving the solid residue obtained in step (iii) in water and subjecting to gel filtration to remove low molecular weight impurities, and
(v) concentrating the eluent obtained in step (iv) under vacuum concentrator and subjecting to high performance liquid chromatography (HPLC) to obtain the free fully deprotected oligodeoxyribo and oligoribonucleotides.

9. A process as claimed in claim 8 wherein 2'-deoxyribonucleosidephosphoramidites and ribonucleosidephosphor-amidites used for the synthesis of oligodeoxyribo-and ribonucleotides in step (i) carrying conventional protecting groups are selected as follows benzoyl for adenine, benzoyl or acetyl for cytosine and isobutyryl for guanine or labile protecting groups are selected from phenoxyacetyl for adenine and guanine and isobutyryl or acetyl for cytosine; dimethylformamidine (DMF) for adenine, guanine and cytosine; p-tert-butylphenoxyacetyl- for adenine, guanine and cytosine and the like for exocyclic amino functions of nucleic bases.

10. A process as claimed in claim 8 wherein the basic medium used for cleaving synthesized oligonucleotides from the universal polymer support and simultaneous removal of protecting groups is selected from aq. Ammonia, ag. methylamine, ag. ammonia -methylamine (1:1,v/v) mixture.

11. A process as claimed in claim 8 wherein the oligomers are cleaved from the universal polymer support and the protecting groups are simultaneously removed at a temperature in the range of 30° -65° C depending upon the protecting groups employed for the protection of exocyclic amino groups of nucleic bases.

12. A process as claimed in claim 8 wherein the time required for cleaving oligomers from the universal polymer support and simultaneous removal of protecting groups is in the range of 5-120 min depending upon the protecting groups employed for the protection of exocyclic amino groups of nucleic bases.

## Patentansprüche

1. Universeller Träger aus Polymer (Vielzweckpolymerträger), der eine organische aliphatische Molekülstruktur der Formel 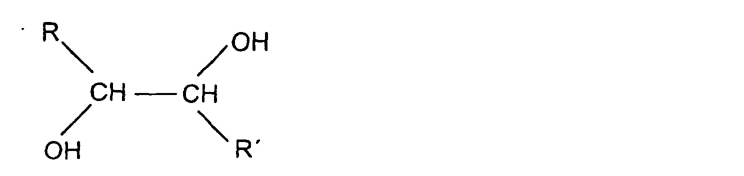 aufweist, worin R = H ― (CH₂)ₙ ― und R' = ― CH₂ ― OH, oder ― (CH₂)ₙ ― H, und n = 1 - 4 bedeuten,
und worin mindestens ein Paar cis-Hydroxylgruppen vorhanden ist, von denen die eine der Hydroxylgruppen über eine kovalente Bindung an den Polymerträger gebunden und die andere Hydroxylgruppe durch eine labile Säuregruppe geschützt ist.

2. Verfahren, das für die Herstellung vollständig von Schutzgruppen befreiter (Desoxyribo - oder Ribo) - Oligonukleotide zweckmäßig ist, das folgende Maßnahmen umfasst:
(i) Behandlung eines aliphatischen organischen Moleküls, das ein Paar cis-Hydroxylgruppen in der Sekundärstellung aufweist, mit 4,4'- Dimethoxytritylchlorid und Isolierung eines monosubstituierten tritylierten Derivats der Formel: 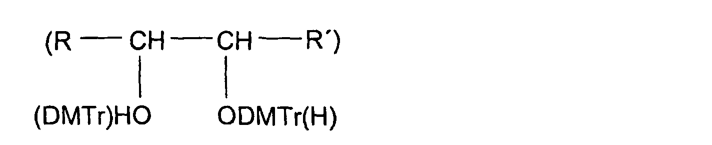 worin R = H ― (CH₂)ₙ ― und R' = ― CH₂ ― OH, oder ― (CH₂)ₙ ― H, und
n = 1 - 4 sowie DMTr = 4,4' - Dimethoxytrityl bedeuten,
(ii) Behandlung der gemäß Schritt (i) gewonnenen monosubstituierten Substanz mit 1 Äquivalent eines homobifunktionellen Halogenids einer Alkan(Carbon)säure und Übertragung des Gemisches auf einen Polymerträger, der funktionelle Hydroxyl- oder Aminoalkylgruppen (-Funktionalitäten) trägt, sowie
(iii) Behandlung des gemäß Schritt (ii) erhaltenen Polymerträgers mit einem getrocknetem Alkanol zum Zwecke des Cappings und nachfolgendes Waschen mit getrocknetem Alkanol bzw. Dialkylether.

3. Verfahren nach Anspruch 2, bei dem das organische aliphatische Molekül mit einem Paar cis-Hydroxylgruppen, das zur Darstellung des monosubstituierten tritylierten Derivats eingesetzt wird, aus der Gruppe ausgewählt wird, die aus 2,3 - Butandiol, 1,2,3 - Trihydroxyheptan und 1,2,3 - Hexantriol besteht.

4. Verfahren nach Anspruch 2, bei dem das in Schritt (ii) verwendete homobifunktionelle Halogenid einer Alkan(Carbon)-Säure aus der Gruppe ausgewählt wird, die aus Oxalylchlorid, Bernsteinsäurechlorid, Adipinsäurechlorid und dergleichen besteht.

5. Verfahren nach Anspruch 2, bei dem der in Schritt (ii) eingesetzte Polymerträger aus organischen oder anorganischen Polymeren ausgewählt wird, zum Beispiel als gesteuertes Porenglas (CPG) mit variabler Porengröße und Linker-Abzweigung, Silikagel (porös oder nicht-porös), quer vernetztes Polystyrol mit funktionellen Hydroxyl- oder Aminoalkylgruppen und dergleichen.

6. Verfahren nach Anspruch 2, bei dem das in Schritt (iii) eingesetzte Alkanol zum Zwecke des Cappings aus Methanol, Ethanol und dergleichen ausgewählt wird.

7. Verfahren zur Herstellung von Oligonukleotiden (sowohl Oligodesoxyribo- als auch Oligoribonukleotiden), bei dem ein verbesserter Vielzweckpolymerträger nach Anspruch 1 verwendet wird, der mit den bereits vorhandenen Verfahren der Oligonukleotidsynthese und der Entfernung von Schutzgruppen unter Verwendung von Nukleosidphosphoramidit - Retrosyntheseprodukten kompatibel ist, wobei letztere herkömmliche oder basische labile Schutzgruppen zum Schützen von exocyclischen funktionellen Aminogruppen der Nukleinbasen tragen.

8. Verfahren nach Anspruch 7, bei dem die Herstellung von Oligonukleotiden unter Einsatz eines verbesserten Vielzweckträgers die folgenden Maßnahmen umfasst:
(i) Synthese von Oligodesoxyribo- und Oligoribonukleotiden an einem verbesserten Vielzweckpolymerträger gemäß der Phosphoramiditchemie unter Einsatz von herkömmlichen oder labilen Schutzgruppen für Nukleinbasen,
(ii) Behandeln der in dem Träger eingebetteten Oligodesoxynukleotide und Oligoribonukleotide mit einem basischen Medium,
(iii) Einengen des im Schritt (ii) gewonnenen Reaktionsrückstandes in einem Vakuumkonzentrator,
(iv) Auflösen des im Schritt (iii) gewonnenen festen Rückstands in Wasser und seine Gelfiltration zum Entfernen von Verunreinigungen niedrigen Molekulargewichts sowie
(v) Einengen des im Schritt (iv) erhaltenen Eluats im Vakuumkonzentrator und seine Höchstleistungsflüssigkeitschromatographie (HPLC) zum Erzielen der freien, vollständig von Schutzgruppen befreiten Oligodesoxyribo- und Oligoribonukleotide.

9. Verfahren nach Anspruch 8, bei dem 2'- Desoxyribonukleosidphosphoramidite und Ribonukleosidphosphoramidite, die für die Synthese von Oligodesoxyribo- und Ribonukleotiden im Schritt (i) eingesetzt werden und welche herkömmliche Schutzgruppen tragen, ausgewählt werden wie folgt: Benzoyl für Adenin, Benzoyl oder Acetyl für Cytosin und Isobutyryl für Guanin oder labile Schutzgruppen werden ausgewählt aus Phenoxyacetyl für Adenin und Guanin und Isobutyryl oder Acetyl für Cytosin; Dimethylformamidin (DMF) für Adenin, Guanin und Cytosin; p-tert-Butylphenoxyacetyl- für Adenin, Guanin und Cytosin und dergleichen für exocyclische funktionelle Aminogruppen von Nukleinbasen.

10. Verfahren nach Anspruch 8, bei dem das zum Abspalten der synthetisierten Oligonukleotide vom Vielzweckpolymerträger und für die gleichzeitige Entfernung der Schutzgruppen verwendete basische Medium ausgewählt wird aus wässrigem Ammoniak, wssrg. Methylamin und einem wssrg. Gemisch aus Ammoniak und Methylamin im Verhältnis von 1: 1 (Vol./Vol.).

11. Verfahren nach Anspruch 8, bei dem die Oligomere von dem Vielzweckpolymerträger abgespalten und gleichzeitig die Schutzgruppen entfernt werden bei einer Temperatur im Bereich von 30° bis 65 °C, abhängig von den zum Schützen der exocyclischen Aminogruppen von Nukleinbasen eingesetzten Schutzgruppen.

12. Verfahren nach Anspruch 8, bei dem die zum Abspalten von Oligomeren von dem Vielzweckträger und gleichzeitigem Entfernen der Schutzgruppen benötigte Zeit im Bereich von 5 bis 120 Minuten liegt, je nach den zum Schützen der exocyclischen Aminogruppen von Nukleinbasen eingesetzten Schutzgruppen.

## Revendications

1. Support polymère universel contenant une structure de molécule aliphatique organique 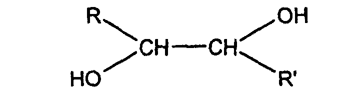 R = H-(CH₂)ₙ- et
R' = -CH₂-OH, -(CH₂)ₙ-H ;
n = 1-4 ;
ayant au moins une paire de groupes cis-hydroxyle où l'un des groupes hydroxyle est rattaché au support polymère par l'intermédiaire d'une liaison covalente et l'autre groupe hydroxyle est protégé par un groupe labile aux acides.

2. Procédé utiles pour la préparation d'oligonucléotides (désoxyribo- ou ribo) complètement déprotégés, qui comprend :
(i) le traitement d'une molécule organique aliphatique ayant une paire de groupes cis-hydroxyle en position secondaire avec du chlorure de 4,4'-diméthoxytrityle et l'isolation d'un dérivé tritylé monosubstitué de formule 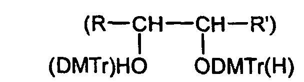 où R = H-(CH₂)ₙ et R' = -CH₂OH, -(CH₂)ₙ-H ; n = 1-4 ; DMTr = 4,4'-diméthoxytrityle,
(ii) le traitement de la substance monosubstituée obtenue dans l'étape (i) avec un équivalent d'un halogénure d'acide alcanoïque homobifonctionnel et le transfert du mélange sur un support polymère portant des fonctionnalités hydroxyle ou aminoalkyle, et
(iii) le traitement du support polymère obtenu dans l'étape (ii) avec un alcanol sec dans un but de coiffage, suivi d'un lavage avec un alcanol sec et un dialkyléther, respectivement.

3. Procédé selon la revendication 2, dans lequel la molécule aliphatique organique ayant une paire de groupes cis-hydroxyle, utilisée pour préparer le dérivé tritylé monosubstitué, est choisie dans l'ensemble constitué par le butane-2,3-diol, le 1,2,3-trihydroxyheptane et le 1,2,3-hexanetriol.

4. Procédé selon la revendication 2, dans lequel l'halogénure d'acide alcanoïque homobifonctionnel utilisé dans l'étape (ii) est choisi dans l'ensemble constitué par le chlorure d'oxalyle, le chlorure de succinoyle, le chlorure d'adipoyle et analogues.

5. Procédé selon la revendication 2, dans lequel le support polymère employé dans l'étape (ii) est choisi parmi les polymères organiques et minéraux, par exemple le verre à pores contrôlés (CPG) ayant des tailles de pores et des bras lieurs variables, le gel de silice (poreux ou non poreux), le polystyrène réticulé ayant des fonctions hydroxyle ou aminoalkyle, et analogues.

6. Procédé selon la revendication 2, dans lequel l'alcanol utilisé dans l'étape (iii) à des fins de coiffage est choisi parmi le méthanol, l'éthanol et analogues.

7. Procédé pour la préparation d'oligonucléotides (tant oligodésoxyribo- qu'oligoribo-) utilisant un support polymère universel amélioré selon la revendication 1 compatible avec le procédé existant de synthèse et de déprotection des oligonucléotides utilisant des synthons de nucléoside-phosphoramidite portant des groupes protecteurs conventionnels ou labiles aux acides pour la protection de fonctionnalités amino exocycliques des bases nucléiques.

8. Procédé selon la revendication 7, dans lequel la préparation d'oligonucléotides utilisant un support universel amélioré comprend :
(i) la synthèse d'oligodésoxyribo- et oligoribonucléotides sur un support polymère universel amélioré après chimie des phosphoramidites employant des protecteurs conventionnels ou labiles pour les bases nucléiques,
(ii) le traitement de l'oligodésoxynucléotide et des oligoribonucléotides noyés dans le support avec un milieu basique,
(iii) la concentration du résidu réactionnel obtenu dans l'étape (ii) dans un concentrateur sous vide,
(iv) la dissolution du résidu solide obtenu dans l'étape
(iii) dans de l'eau et la soumission à une filtration sur gel pour éliminer les impuretés de faible masse moléculaire, et
(v) la concentration de l'éluant obtenu dans l'étape (iv) dans le concentrateur sous vide et la soumission à une chromatographie liquide haute performance (CLHP) pour obtenir les oligodésoxyribo- et oligoribo-nucléotides libres complètement déprotégés.

9. Procédé selon la revendication 8, dans lequel les 2'-désoxyribonucléoside-phosphoramidites et ribonucléoside-phosphoramidites utilisés pour la synthèse d'oligodésoxyribo- et ribo-nucléotides dans l'étape (i) portant des groupes protecteurs conventionnels sont choisis comme suit : benzoyle pour l'adénine, benzoyle ou acétyle pour la cytosine, et isobutyryle pour la guanine, ou bien les groupes protecteurs labiles sont choisis parmi les suivants : phénoxyacétyle pour l'adénine et la guanine et isobutyryle ou acétyle pour la cytosine ; diméthylformamidine (DMF) pour l'adénine, la guanine et la cytosine ; p-tert-butylphénoxyacétyle pour l'adénine, la guanine et la cytosine et analogues pour les fonctions amino exocycliques de bases nucléiques.

10. Procédé selon la revendication 8, dans lequel le milieu basique utilisé pour couper les oligonucléotides synthétiques d'avec le support polymère universel et éliminer simultanément les groupes protecteurs est choisi parmi l'ammoniaque, une solution aqueuse de méthylamine, et un mélange d'ammoniaque et de solution aqueuse de méthylamine 1/1 en volume.

11. Procédé selon la revendication 8, dans lequel les oligomères sont coupés du support polymère universel et les groupes protecteurs sont simultanément éliminés à une température située dans la plage allant de 30 à 65°C en fonction des groupes protecteurs employés pour la protection de groupes amino exocycliques de bases nucléiques.

12. Procédé selon la revendication 8, dans lequel le temps requis pour couper les oligomères du support polymère universel et simultanément éliminer les groupes protecteurs est situé dans la plage allant de 5 à 120 minutes en fonction des groupes protecteurs employés pour la protection de groupes amino exocycliques de bases nucléiques.
